(19) Europäisches Patentamt
European Patent Office
Office européen des brevets

(11) **EP 1 624 030 B1**

(12) **EUROPÄISCHE PATENTSCHRIFT**

(45) Veröffentlichungstag und Bekanntmachung des Hinweises auf die Patenterteilung:
**02.02.2011  Patentblatt 2011/05**

(51) Int Cl.:
***C09C 1/00*** *(2006.01)*

(21) Anmeldenummer: **05014604.2**

(22) Anmeldetag: **06.07.2005**

(54) **Mischung aus Interferenzpigmenten**

Mixture of interference pigments

Mélange de pigments d'interférence

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **27.07.2004  DE 102004036297**

(43) Veröffentlichungstag der Anmeldung:
**08.02.2006  Patentblatt 2006/06**

(73) Patentinhaber: **Merck Patent GmbH 64293 Darmstadt (DE)**

(72) Erfinder: **Kniess, Helge Bettina 64331 Weiterstadt (DE)**

(56) Entgegenhaltungen:
**EP-A- 1 213 330      EP-A- 1 422 268 DE-A1- 10 331 903**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 624 030 B1

## Beschreibung

Die vorliegende Erfindung betrifft eine silberne und farbneutrale Pigmentmischung umfassend farbschwache silberne Interterenzpigmente A und farbschwache komplementär-farbige Interferenzpigmente B, deren Verwendung in Kosmetika, Lacken, Farben, Kunststoffen, Folien, im Sicherheitsdruck, in Sicherheitsmerkmalen in Dokumenten und Ausweisen, zur Saatguteinfärbung, zur Lebensmitteleinfärbung oder in Arzneimittelüberzügen und zur Herstellung von Pigmentpräparationen und Trockenpräparaten sowie Kosmetika, Lacke, Farben, Kunststoffe, Folien, Sicherheitsmerlcmale in Dokumenten und Ausweisen, Saatgut, Lebensmittel- oder Arzneimittelüberzüge sowie Pigmentpräparationen und Trockenpräparate enthaltend die erfindungsgemäße Pigmentmischung.

Silberfarbene Gegenstände finden sich in allen Bereichen des täglichen Bedarfs. Dies gilt insbesondere für Automobile, aber auch für viele andere Gegenstände, z.B. aus Kunststoffen. Darüber hinaus ist ein silbernes Erscheinungsbild auch bei Anwendung von Lacken, z.B. bei Gebäuden und dergleichen gefragt. Neben den klassischen Metalleffektpigmenten sind auch Interferenzpigmente, wie sie in EP 1213330 A, DE 10331903 A und EP 1422208 A beschrieben sind, geeignet, diesen optischen Eindruck zu erzeugen. Anders als bei den Metalleffektpigmenten, bei denen der optische Effekt auf der Reflexion an der Oberfläche des metallischen Kems basiert, beruht der optische Eindruck der Interferenzpigmente gemäß DIN 55944 auf dem Phänomen der Licht-Interferenz, wie beispielsweise bei Pigmenten umfassend Titandioxid auf Glimmer. Die Interferenzpigmente haben gegenüber den Metalleffektpigmenten den Vorteil, dass sie durch die entsprechende Auswahl der Substratkerne und der drauf aufgebrachten Beschichtung variabel bezüglich ihrer Transparenz sind, was bei Metalleffektpigmenten wegen des grundsätzlichen deckenden Metallkemes nicht möglich ist. Durch die einstellbare Transparenz der Interferenzpigmente werden dem Anwender zusätzliche Gestaltungsoptionen ermöglicht, z.B. durch die Kombination mit farbgebenden Systemen, die sich in den jeweiligen Anwendungen auch unterhalb der Interferenzpigmente enthaltenden Schicht befinden können. Dies gilt insbesondere für den Automobilbereich, wo der Gesamteindruck des Lackes durch die Kombination verschiedener optischer Effekte in den einzelnen Lackschichten erzielt wird.

Üblicherweise lassen sich mit mehrschichtigen Interferenzpigmenten nur schwer farbneutrale Silbertöne erzielen, das heißt, in der Regel zeigen silberne Interferenzpigmente einen Farbstich, z.B. eine schwache blaue oder grünliche Färbung. Man kann einen solchen Farbstich durch die Zumischung einer gewissen Menge eines Pigmentes komplementärer Farbe ausgleichen. Dies führt jedoch zu einem Glanzverlust, der bei Silberpigmenten aber gerade besonders nachteilig ist, da hier ein besonders hoher Glanz erwünscht ist. Um dem entgegen zu wirken, kann man ein möglichst farbstarkes komplementär-farbiges Pigment zur Abmischung verwenden, um auf diese Weise die zur Neutralisierung des Farbstichs erforderliche Menge zu minimieren. Der Einsatz eines farbstarken Pigmentes erfordert jedoch eine hohe Präzision bei der Abmischung, um nicht den gegenteiligen Effekt einer Färbung in Richtung der Komplementärfarbe zur erhalten. Darüber hinaus sind bei einer Mischung aus farbstarken und koloristisch deutlich unterschiedlichen Pigmenten die einzelnen Pigmentpartikel häufig als separierte Farbpunkte wahrnehmbar. Das ursprünglich homogene Erscheinungsbild wirkt dadurch unstetig und unruhig. Dieser visuelle Eindruck verstärkt sich zusätzlich noch mit zunehmender Partikelgröße.

Es bestand daher die Aufgabe, einen farbneutralen silbernen Effekt zu erreichen, der für den Anwender in einfacher Weise zugänglich ist, ohne dabei den Glanz oder das Erscheinungsbild in der Anwendung negativ zu beeinflussen.

Diese Aufgabe wird durch Pigmentmischungen gemäß der vorliegenden Erfindung erfüllt. Gegenstand der vorliegenden Erfindung ist demnach eine silberne und farbneutrale Pigmentmischung gemäß Anspruch 1 umfassend farbschwache silberne Interferenzpigmente A und farbschwache komplementär-farbige Interferenzpigmente B.

Pigmentmischungen gemäß der vorliegenden Erfindung zeichnen sich durch einen farbneutralen silbernen Eindruck aus, wobei der Glanz nicht wesentlich beeinträchtigt ist. Durch die Verwendung eines farbschwachen komplementär-farbigen Interferenzpigmerites wird die Abmischung vereinfacht, weil die Toleranz in bezug auf die Menge des eingesetzten Pigmentes größer ist. Überraschenderweise wurde hierbei gefunden, dass die tatsächlich einzusetzende Menge des farbschwachen komplementär-farbigen Interferenzpigments B derjenigen eines entsprechenden farbstarken Pigmentes entspricht. Insgesamt ist damit der Glanz und der erreichbare Effekt bei Pigmentmischungen gemäß der vorliegenden Erfindung damit größer als bei Pigmentmischungen aus dem Stand der Technik.

Sowohl die farbschwachen silbernen Interferenzpigmente A als auch die farbschwachen komplementär-farbigen Interferenzpigmente B können einen mehrschichtigen Aufbau aufweisen. Daher besteht die Möglichkeit eine Mischung herzustellen, die ausschließlich aus mehrschichtigen interferenzpigmenten besteht. Auf diese Weise werden die anwendungstechnischen Eigenschaften und Vorteile der Mehrschichtpigmente, wie z.B. eine verbesserte Reinigungsfähigkeit damit pigmentierter Pulveriackbeschichtungen, erhalten.

Wegen der oben genannten Vorteile sind die erfindungsgemäßen Pigmentmischungen vielseitig einsetzbar, Gegenstand der vorliegenden Erfindung sind demgemäss auch die Verwendung der Pigmentmischungen in Kosmetika, Lacken, Farben, Kunststoffen, Folien, im Sicherheitsdruck, in Sicherheitsmerkmalen in Dokumenten und Ausweisen, zur Saatguteinfärbung, zur Lebensmitteleinfärbung oder in Arzneimittelüberzügen und zur Herstellung von Pigmentpräparationen und Trockenpräparaten sowie Kosmetika, Lacke, Farben, Kunststoffe, Folien, Dokumente und Ausweise, Saatgut, Lebensmittel oder Arzneimittelüberzüge Pigmentpräparationen und Trockenpräparate enthaltend Pigmentmischungen gemäß der vorliegenden Erfindung.

In der erfindungsgemäßen Pigmentmischung werden farbschwache silberne Interferenzpigmente A und farbschwache komplementär-farbige Interferenzpigmente B miteinander kombiniert, um eine farbneutrale silberne Mischung mit hohem Glanz zu erreichen. Die Farbstärke C lässt sich aus gemessenen L,a,b-Werten ableiten (siehe, F. Hofmeister, Colorimetric evaluation of pearlescent pigments, Congress "Mondial Couleur 85", Monte Carlo, 1985). Der Wert für die Farbstärke C ergibt sich aus den Werten für a und b gemäß:

$$C = \sqrt{a^2_{22.5°/22.5° \, Schwarzkarte} + b^2_{22.5°/22.5° \, Schwarzkarte}}$$

Im Rahmen der vorliegenden Erfindung ist der Wert für die Farbstärke C bei farbstarken Pigmenten > 10.0, bei farbschwachen Pigmenten liegt der Wert für die Farbstärke C zwischen 4.0 und 10.0 und als Farbneutralität wird definiert, wenn der Wert für die Farbstärke C ≤ 4.0 ist. Der Wert C für die Farbstärke liegt bei den farbschwachen Interferenzpigmenten A und B demnach zwischen 4.0 und 10.0 und der Wert C für die Farbstärke der erfindungsgemäßen Pigmentmischung ist ≤ 4.0.

Prinzipiell können die Interferenzpigmente A und B jede denkbare Form aufweisen, vorzugsweise sind sie aber plättchenförmig. Die Größe der Interferenzpigmente ist an sich nicht kritisch. Plättchenförmige Interferenzpigmente weisen in der Regel eine Dicke zwischen 0.05 und 5 μm, insbesondere zwischen 0.1 und 4.5 μm auf. Die Ausdehnung der Interferenzpigmente in der Länge bzw. Breite kann zwischen 1 und 250 μm betragen, vorzugsweise liegt sie im Bereich von 2 bis 200 μm und ganz besonders bevorzugt im Bereich von 2 bis 100 μm. Die Größe der Interferenzpigmente kann an die Anforderungen der jeweiligen Applikationen angepasst werden.

Die Interferenzpigmente A und B basieren auf Trägermaterialien, die mit einer oder mehreren Schichten enthaltend Metalloxide, Metalloxidhydrate, Metallsuboxide, Metalle, Metallfluoride, Metallnitride, Metalloxynitride und/oder Mischungen hieraus beschichtet sind. Das Trägermaterial kann Titanoxide, synthetischen oder natürlichen Glimmer, Schichtsilikate, Glas, Siliziumdioxid, Eisenoxid und/oder Aluminiumoxid umfassen, vorzugsweise handelt es sich um synthetischen oder natürlichen Glimmer, um Glas, Siliziumdioxid oder Aluminiumoxid. Die Beschichtung mit einer oder mehreren Schichten enthaltend Metalloxide, Metalloxidhydrate, Metallsuboxide, Metalle, Metallfluoride, Metallnitride, Metalloxynitride und/oder Mischungen hieraus bestimmt maßgeblich den optischen Eindruck der Interferenzpigmente. Die Metalloxid-, Metalloxidhydrat, Metallsuboxid-, Metall-, Metallfluorid-, Metallnitrid-, Metalloxynitridschichten oder die Mischungen hieraus können niedrig- (Brechzahl < 1.8) oder hochbrechend (Brechzahl ≥ 1.8) sein. Als Metalloxide und Metalloxidhydrate eignen sich alle als Schichten aufzubringende Metalloxide oder Metalloxidhydrate, wie z.B. Aluminiumoxid, Aluminiumoxidhydrat, Eisenoxid, Zinnoxid, Ceroxid, Zinkoxid, Zirkoniumoxid, Chromoxid, Titanoxid, insbesondere Titandioxid, Titanoxidhydrat sowie Mischungen hieraus, wie z.B. Ilmenit oder Pseudobrookit. Als Metallsuboxide können beispielsweise die Titansuboxide eingesetzt werden. Als Metalle eignen sich z.B. Chrom, Aluminium, Nickel, Silber, Gold, Titan, Kupfer oder Legierungen, als Metallfluorid eignet sich beispielsweise Magnesiumfluorid. Als Metallnitride oder Metalloxynitride können beispielsweise die Nitride oder Oxynitride der Metalle Titan, Zirkonium und/oder Tantal eingesetzt werden. Bevorzugt sind Metalloxid-, Metall-, Metallfluorid und/oder Metalloxidhydratschichten und ganz besonders bevorzugt Metalloxid- und/oder Metalloxidhydratschichten auf den Trägermaterialien aufgebracht. Weiterhin können auch Mehrschichtaufbauten aus hoch- und niedrigbrechenden Metalloxid-, Metalloxidhydrat-, Metall- oder Metallfluoridschichten vorliegen, wobei sich vorzugsweise hoch- und niedrigbrechende Schichten abwechseln.

In einer bevorzugten Ausführungsform sind die genannten Trägermaterialien bei den Interferenzpigmenten A und B mit alternierenden Schichten von Materialien mit hoher und niedriger Brechzahl beschichtet.

Besonders geeignete Materialien mit hoher Brechzahl sind beispielsweise $TiO_2$, $ZrO_2$, ZnO, $SnO_2$, BiOCl und/ oder Mischungen hieraus. Besonders bevorzugt ist $TiO_2$. Die Dicke dieser Schichten beträgt dabei jeweils etwa 3 bis 300 nm und bevorzugt 20 bis 200 nm.

Besonders geeignete Materialien mit niedriger Brechzahl sind beispielsweise $SiO_2$, $SiO(OH)_2$, $Al_2O_3$, AlO(OH), $B_2O_3$, $MgF_2$ und/oder Mischungen hieraus. Besonders bevorzugt ist $SiO_2$. Die Dicke der einzelnen Schichten aus diesen Materialien beträgt zwischen 3 und 300 nm, bevorzugt 20 bis 200 nm.

Die äußere Schicht der einsetzbaren Interferenzpigmente A und B besteht vorzugsweise aus einem hochbrechenden Material, insbesondere aus $TiO_2$. Ausgehend von dieser Bedingung ergeben sich für den bevorzugten Aufbau der Interferenzpigmente A und B bestimmte Gesetzmäßigkeiten. Besteht das Substrat aus einem hochbrechenden Material, wie z.B. $TiO_2$ oder Eisenoxid, so erfolgt eine Belegung mit einer Schicht eines Materials mit niedriger Brechzahl und einer Schicht eines Materials mit hoher Brechzahl. Im Rahmen der vorliegenden Erfindung wird dabei davon ausgegangen, dass die Belegung vorzugsweise umhüllend erfolgt, das heißt, sowohl das Substrat als auch alle nachfolgenden belegten Zwischenstufen ist in jedem Beschichtungsschritt auf jeder Seite der Plättchen beschichtet. Vorzugsweise handelt es sich bei dem Substratmaterial um ein Material mit niedriger Brechzahl, insbesondere um Glimmer oder $SiO_2$. In diesem Falle erfüllen Pigmente mit drei und sieben Schichten, einschließlich des Trägermaterials, die Bedingung, dass die äußere Schicht aus einem hochbrechenden Material ist. Vorzugsweise umfassen die einsetzbaren Interferenzpigmente insgesamt sieben Schichten, einschließlich des Trägermaterials.

Im Fall der oben genannten Interferenzpigmente A und B mit insgesamt sieben Schichten, liegen insgesamt zwei umhüllende Schichten aus Materialien mit hoher Brechzahl vor. Diese Schichten können aus gleichen oder verschiedenen Materialien bestehen und gleiche oder verschiedene Schichtdicken aufweisen. Vorzugsweise bestehen sie aus gleichen Materialien, insbesondere aus $TiO_2$. In der vorliegenden Erfindung ganz besonders bevorzugt einzusetzende Interferenzpigmente A und B weisen demgemäss folgenden Aufbau auf:

$$TiO_2/SiO_2/TiO_2/Trägermaterial(Glimmer\ oder\ SiO_2)/TiO_2/SiO_2/TiO_2$$

Es unterliegt dem Fachwissen des Fachmanns, die oben genannten Materialien und Schichtdicken derart auszuwählen, dass silberne farbschwache Interferenzpigmente A und farbschwache komplementär-farbige Interferenzpigmente B erhalten werden. Pigmente der oben beschriebenen Art sind bekannt und kommerziell erhältlich, beispielsweise unter den Handelsnamen Iriodin®, Xirallic®, Colorstream® oder Timiron® bei der Fa. Merck KGaA, Darmstadt, sowie bei weiteren Anbietern.

In einer weiteren Ausführungsform der vorliegenden Erfindung können die Interferenzpigmente A und B weiterhin mit einer zusätzlichen organischen Beschichtung als äußere Schicht versehen sein. Beispiele für derartige Beschichtungen finden sich z.B. in EP 0 632 109, US 5,759,255, DE 43 17 019, DE 39 29 423, DE 32 35 017, EP 0 492 223, EP 0 342 533, EP 0 268 918, EP 0 141 174, EP 0 764 191, WO 98/13426 oder EP 0 465 805, deren Offenbarung hiermit unter Bezugnahme mit eingeschlossen ist. Interferenzpigmente enthaltend diese organische Beschichtung, z.B. aus Organosilanen oder Organotitanaten bzw. Organozirkonaten zeigen neben den bereits genannten verbesserten optischen Eigenschaften zusätzlich eine erhöhte Stabilität gegenüber Witterungseinflüssen, wie z.B. Feuchtigkeit und Licht, was vor allem für Industrielacke und im Automobilbereich von besonderem Interesse ist.

Die Herstellung der erfindungsgemäßen Pigmentmischung erfolgt durch Mischen der Interferenzpigmente A und B, wobei die Anteile der Interferenzpigmente A und B derart gewählt werden, dass der Wert C für die Farbstärke der Mischung ≤ 4.0 wird. Erst ab diesem Punkt liegt eine farbneutrale silberne Mischung vor. Der Vorgang des Mischens als solches kann auf alle dem Fachmann bekannte Arten erfolgen.

Die erfindungsgemäßen Pigmentmischungen eignen sich aufgrund ihrer vorteilhaften Eigenschaften für eine große Bandbreite an Anwendungen. Gegenstand der Erfindung ist daher auch die Verwendung der erfindungsgemäßen Pigmentmischungen in Kosmetika, Lacken, Farben, Kunststoffen, Folien, im Sicherheitsdruck, in Sicherheitsmerkmalen in Dokumenten und Ausweisen, zur Lasermarkierung, zur Saatguteinfärbung, zur Lebensmitteleinfärbung oder in Arzneimittelüberzügen sowie zur Herstellung von Pigmentpräparationen und Trockenpräparaten.

Im Falle von Kosmetika eignen sich die erfindungsgemäßen Pigmentmischungen besonders für Produkte der dekorativen Kosmetik, wie z.B. Nagellacke, farbgebende Puder, Lippenstifte oder Lidschatten. Selbstverständlich können die erfindungsgemäßen Pigmentmischungen in den Formulierungen auch mit jeder Art von kosmetischen Roh- und Hilfsstoffen kombiniert werden. Dazu gehören u.a. Öle, Fette, Wachse, Filmbildner, Konservierungsmittel und allgemein anwendungstechnische Eigenschaften bestimmende Hilfsstoffe, wie z.B. Verdicker und rheologische Zusatzstoffe wie etwa Bentonite, Hektorite, Siliziumdioxid, Ca-Silikate, Gelatine, hochmolekulare Kohlenhydrate und/oder oberflächenaktive Hilfsmittel, etc. Die erfindungsgemäßen Pigmentmischungen enthaltenden Formulierungen können dem lipophilen, hydrophilen oder hydrophoben Typ angehören. Bei heterogenen Formulierungen mit diskreten wässrigen und nichtwässrigen Phasen können die erfindungsgemäßen Partikel in jeweils nur einer der beiden Phasen enthalten oder auch über beide Phasen verteilt sein.

Die pH-Werte der wässrigen Formulierungen können zwischen 1 und 14, bevorzugt zwischen 2 und 11 und besonders bevorzugt zwischen 5 und 8 liegen. Den Konzentrationen der erfindungsgemäßen Pigmentmischungen in der Formulierung sind keine Grenzen gesetzt. Sie können - je nach Anwendungsfall - zwischen 0,001 (rinse-off-Produkte, z.B. Duschgele) - 99% (z.B. Glanzeffekt-Artikel für besondere Anwendungen) liegen. Die erfindungsgemäßen Pigmentmischungen können weiterhin auch mit kosmetischen Wirkstoffen kombiniert werden. Geeignete Wirkstoffe sind z.B. Insect Repellents, UV A/BC-Schutzfilter (z.B. OMC, B3, MBC), Anti-Ageing-Wirkstoffe, Vitamine und deren Derivate (z.B. Vitamin A, C, E etc.), Selbstbräuner (z.B. DHA, Erythrolose u.a.) sowie weitere kosmetische Wirkstoffe wie z.B. Bisabolol, LPO, Ectoin, Emblica, Allantoin, Bioflavanoide und deren Derivate.

Bei Einsatz der Pigmentmischungen in Lacken und Farben sind alle dem Fachmann bekannten Anwendungsbereiche möglich, wie z.B. Pulverlacke, Automobillacke, Druckfarben für den Tief-, Offset-, Sieb- oder Flexodruck sowie für Lacke in Außenanwendungen. Die Lacke und Farben können hierbei beispielsweise strahlungshärtend, physikalisch trocknend oder chemisch härtend sein. Für die Herstellung der Druckfarben oder Flüssiglacke ist eine Vielzahl von Bindern, z.B. auf der Basis von Acrylaten, Methacrylaten, Polyestern, Polyurethanen, Nitrocellulose, Ethylcellulose, Polyamid, Polyvinylbutyrat, Phenolharzen, Maleinharzen, Stärke oder Polyvinylalkohol, Aminharzen, Alkydharzen, Epoxidharzen, Polytetrafluorethylen, Polyvinylidenfluoriden, Polyvinylchlorid oder Mischungen hieraus geeignet, insbesondere wasserlösliche Typen. Bei den Lacken kann es sich um Pulverlacke oder wasser- oder lösemittelbasierte Lacke handeln, wobei die Auswahl der Lackbestandteile dem Allgemeinwissen des Fachmanns unterliegt. Gängige polymere Bindemittel für Pulverlacke sind beispielsweise Polyester, Epoxide, Polyurethane, Acrylate oder Mischungen hieraus.

Darüber hinaus können die erfindungsgemäßen Pigmentmischungen zur Pigmentierung von Folien und Kunststoffen verwendet werden, so z.B. für Agrarfolien, Geschenkfolien, Kunststoffbehältnisse und Formkörper für alle dem Fachmann bekannten Anwendungen. Als Kunststoffe eignen sich alle gängigen Kunststoffe für die Einarbeitung der erfindungsgemäßen Pigmentmischungen, z.B. Duromere oder thermoplastische Kunststoffe. Die Beschreibung der Anwendungsmöglichkeiten und der einsetzbaren Kunststoffe, Verarbeitungsverfahren und Additive finden sich z.B. in der RD 472005 oder in R. Glausch, M. Kieser, R. Maisch, G. Pfaff, J. Weitzel, Perlglanzpigmente, Curt R. Vincentz Verlag, 1996, 83 ff., deren Offenbarungsgehalt hier mit umfasst ist.

Darüber hinaus eignen sich die erfindungsgemäßen Pigmentmischungen auch für den Einsatz im Sicherheitsdruck und in sicherheitsrelevanten Merkmalen für z.B. fälschungssichere Karten und Ausweise, wie z.B. Eintrittskarten, Personalausweise, Geldscheine, Schecks und Scheckkarten sowie für andere fälschungssichere Dokumente. Im Bereich der Landwirtschaft können die Pigmentmischungen zur Einfärbung von Saatgut und anderen Ausgangsgütern verwendet werden, darüber hinaus im Lebensmittelbereich zur Pigmentierung von Lebensmitteln. Zur Pigmentierung von Überzügen in Arzneimitteln wie z.B. Tabletten oder Dragees sind die erfindungsgemäßen Pigmentmischungen ebenfalls einsetzbar.

Die erfindungsgemäßen Pigmentmischungen eignen sich ebenfalls zur Pigmentierung von Folien, sowie besonders zur Verwendung in Abmischungen mit allen bekannten organischen und/oder anorganischen Farbmitteln, wie z.B. organischen Farbstoffen, organischen Pigmenten, anorganischen Ein- oder Mehrschichtpigmenten, anorganischen Farbstoffen oder Pigmenten. Damit lassen sich auf einfache Weise neuartige Farbeffekte erzielen, die mit den herkömmlichen Pigmenten auf Metallbasis nur schwer erreichbar sind. Die erfindungsgemäßen Pigmentmischungen können in jedem Verhältnis mit handelsüblichen Pigmenten und Füllern gemischt werden.

Als Füllstoffe sind z.B. natürlicher und synthetischer Glimmer, Nylon Powder, reine oder gefüllte Melaninharze, Talcum, Gläser, Kaolin, Oxide oder Hydroxide von Aluminium, Magnesium, Calcium, Zink, BiOCl, Bariumsulfat, Calciumsulfat, Calciumcarbonat, Magnesiumcarbonat, Kohlenstoff, sowie physikalische oder chemische Kombinationen dieser Stoffe zu nennen. Bezüglich der Partikelform des Füllstoffes gibt es keine Einschränkungen; sie kann den Anforderungen gemäß z.B. plättchenförmig, sphärisch oder nadelförmig sein.

Die erfindungsgemäßen Pigmentmischungen sind weiterhin geeignet zur Herstellung von fließfähigen Pigmentpräparationen und Trockenpräparaten enthaltend ein oder mehrere erfindungsgemäße Partikel, Bindemittel und optional ein oder mehrere Additive. Unter Trockenpräparate sind auch Präparate zu verstehen, die 0 bis 8 Gew.-%, vorzugsweise 2 bis 8 Gew.-%, insbesondere 3 bis 6 Gew.-%, an Wasser und/oder eines Lösemittels oder Lösemittelgemisches enthalten. Die Trockenpräparate liegen vorzugsweise als Pellets, Granulate, Chips, Würstchen oder Briketts vor und weisen Teilchengrößen von 0.2-80 mm auf. Die Trockenpräparate finden insbesondere Anwendung bei der Herstellung von Druckfarben und in kosmetischen Formulierungen.

Kosmetika, Lacke, Farben, Kunststoffe, Folien, Dokumente und Ausweise, Saatgut, Lebensmittel oder Arzneimittelüberzüge Pigmentpräparationen und Trockenpräparate enthaltend die erfindungsgemäßen Pigmentmischungen sind ebenfalls Gegenstand der vorliegenden Erfindung.

Die nachfolgenden Beispiele sollen die Erfindung näher erläutern, ohne sie jedoch zu begrenzen.

**Beispiele:**

a) Herstellung eines farbschwachen grünlich-silbernen Pigmentes mit der Zusammensetzung: $TiO_2/SiO_2/TiO_2/$Glimmer/ $TiO_2/ SiO_2/TiO_2$

100 g Glimmer der Teilchengröße 10-60 $\mu$m werden in 2 l voll entsalztem Wasser unter Rühren suspendiert und auf 75°C erhitzt. Der pH-Wert der Suspension wird mit 18%-iger Salzsäure auf 1.8 eingestellt. 400 g einer 30%-igen Titantetrachloridlösung (hergestellt durch Lösung von 200 g Titantetrachloridlösung (w=60 Gew.-%) in 200 g voll entsalztem Wasser) werden zudosiert, wobei der pH-Wert durch gleichzeitiges Zutropfen einer 32%-igen Natronlauge konstant gehalten wird. Nach vollständiger Zugabe wird 15 Minuten nachgerührt.

Anschließend wird der pH-Wert der Suspension mit 32%-iger Natronlauge auf 7.5 eingestellt. Danach wird eine Natronwasserglas-Lösung (135 g Natronwasserglas-Lösung enthaltend 27 Gew.-% $SiO_2$, gelöst in 135 g voll entsalztem Wasser) zugetropft, wobei der pH-Wert durch gleichzeitiges Zudosieren einer 18%-igen Salzsäure konstant bei 7.5 gehalten wird. Nach vollständiger Zugabe wird 30 Minuten nachgerührt. Nun wird der pH-Wert der Suspension mit 18%-iger Salzsäure auf 1.8 eingestellt, 30 Minuten nachgerührt und 288 g einer 30%-igen Titantetrachloridlösung zugetropft. Durch Zugabe von 32%-iger Natronlauge wird der pH-Wert bei konstant 1.8 gehalten. Es wird erneut 15 Minuten nachgerührt.

Das Produkt wird abfiltriert, gewaschen, getrocknet, bei 850°C kalziniert und durch ein 100 μm Sieb gesiebt.

Vom Pigment werden nach Einarbeitung in Nitrocellulose-Lack Lackkarten angefertigt und diese koloristisch vermessen (Phyma, Geometrien 45°/0° Schwarzkarte, 22,5°/22,5° Schwarzkarte, 45°/0° Weißkarte).

Die Farbstärke des Pigmentes errechnet sich aus:

$$C = \sqrt{a^2_{22.5°/22.5° \text{ Schwarzkarte}} + b^2_{22.5°/22.5° \text{ Schwarzkarte}}}$$

Als Maß für den Glanz wird der auf der Schwarzkarte gemessene L-Wert (22.5°/22.5° Geometrie) herangezogen.

Das Pigment zeigt eine grünlich silberne Interferenz (Farbstärke C = 6.95) mit sehr hohem Glanz (L-Wert: 88.03).

b) Herstellung eines farbschwachen rötlichen Pigmentes mit der Zusammensetzung: $TiO_2$/$SiO_2$/$TiO_2$/Glimmer/ $TiO_2$/ $SiO_2$/$TiO_2$

100 g Glimmer der Teilchengröße 10-60 μm werden in 2 l voll entsalztem Wasser unter Rühren suspendiert und auf 75°C erhitzt. Der pH-Wert der Suspension wird mit 18%-iger Salzsäure auf 1.8 eingestellt. 400 g einer 30%-igen Titantetrachloridlösung (hergestellt durch Lösung von 200 g Titantetrachloridlösung (w=60 Gew.-%) in 200 g voll entsalztem Wasser) werden zudosiert, wobei der pH-Wert durch gleichzeitiges Zutropfen einer 32%-igen Natronlauge konstant gehalten wird. Nach vollständiger Zugabe wird 15 Minuten nachgerührt.

Anschließend wird der pH-Wert der Suspension mit 32%-iger Natronlauge auf 7.5 eingestellt. Danach wird eine Natronwasserglas-Lösung (135 g Natronwasserglas-Lösung enthaltend 27 Gew.-% $SiO_2$, gelöst in 135 g voll entsalztem Wasser) zugetropft, wobei der pH-Wert durch gleichzeitiges Zudosieren einer 18%-igen Salzsäure konstant bei 7.5 gehalten wird. Nach vollständiger Zugabe wird 30 Minuten nachgerührt. Nun wird der pH-Wert der Suspension mit 18%-iger Salzsäure auf 1.8 eingestellt, 30 Minuten nachgerührt und 800 g einer 30%-igen Titantetrachloridlösung zugetropft. Durch Zugabe von 32%-iger Natronlauge wird der pH-Wert bei konstant 1.8 gehalten. Es wird erneut 15 Minuten nachgerührt.

Das Produkt wird abfiltriert, gewaschen, getrocknet, bei 850°C kalziniert und durch ein 100 μm Sieb gesiebt.

Vom Pigment werden nach Einarbeitung in Nitrocellulose-Lack Lackkarten angefertigt und diese koloristisch vermessen (Phyma, Geometrien 45°/0° Schwarzkarte, 22,5°/22,5° Schwarzkarte, 45°/0° Weißkarte).

Das Pigment zeigt eine schwach rötliche Interferenz (Farbstärke C = 7.07) mit einem für ein Rotpigment sehr hohen Glanz (L-Wert: 59.89).

c) Herstellung eines farbstarken roten Interferenzpigmentes mit der Zusammensetzung: $TiO_2$/Glimmer/$TiO_2$

100 g Glimmer der Teilchengröße 10-60 μm werden in 2 l voll entsalztem Wasser unter Rühren suspendiert und auf 75°C erhitzt. Der pH-Wert der Suspension wird mit 18%-iger Salzsäure auf 1.8 eingestellt. 400 g einer 30%-igen Titantetrachloridlösung (hergestellt durch Lösung von 200 g Titantetrachloridlösung (w=60 Gew.-%) in 200 g voll entsalztem Wasser) werden zudosiert, wobei der pH-Wert durch gleichzeitiges Zutropfen einer 32%-igen Natronlauge konstant gehalten wird. Nach vollständiger Zugabe wird 15 Minuten nachgerührt.

Das Produkt wird abfiltriert, gewaschen, getrocknet, bei 850°C kalziniert und durch ein 100 μm Sieb gesiebt.

Vom Pigment werden nach Einarbeitung in Nitrocellulose-Lack Lackkarten angefertigt und diese koloristisch vermessen (Phyma, Geometrien 45°/0° Schwarzkarte, 22,5°/22,5° Schwarzkarte, 45°/0° Weißkarte).

Das Pigment zeigt eine farbstarke rote Interferenz (Farbstärke C = 18.95) mit geringerem Glanz (L-Wert: 50.95) als das Pigment aus Beispiel b).

d) Herstellung eines farbstarken grünen Interferenzpigmentes mit der Zusammensetzung: $TiO_2$/Glimmer/$TiO_2$

100 g Glimmer der Teilchengröße 10-60 μm werden in 2 l voll entsalztem Wasser unter Rühren suspendiert und auf 75°C erhitzt. Der pH-Wert der Suspension wird mit 18%-iger Salzsäure auf 1.8 eingestellt. 1060 g einer 30%-igen Titantetrachloridlösung (hergestellt durch Lösung von 530 g Titantetrachloridlösung (w=60 Gew.-%) in 530 g voll entsalztem Wasser) werden zudosiert, wobei der pH-Wert durch gleichzeitiges Zutropfen einer 32%-igen Natronlauge konstant gehalten wird. Nach vollständiger Zugabe wird 15 Minuten nachgerührt.

Das Produkt wird abfiltriert, gewaschen, getrocknet, bei 850°C kalziniert und durch ein 100 μm Sieb gesiebt.

Vom Pigment werden nach Einarbeitung in Nitrocellulose-Lack Lackkarten angefertigt und diese koloristisch vermessen (Phyma, Geometrien 45°/0° Schwarzkarte, 22,5°/22,5° Schwarzkarte, 45°/0° Weißkarte).

Das Pigment zeigt eine grüne Interferenz (Farbstärke C = 15.15) mit deutlich geringerem Glanz (L-Wert: 65.67) als das Pigment aus Beispiel a).

Beispiel 1: Herstellung einer farbneutralen silbernen Pigmentmischung

Die Pigmentpulver aus a) und b) werden im Verhältnis 2:1 (grünlich-silber:rötlich) gemischt.
Von der Pigmentmischung werden nach Einarbeitung in Nitrocellulose-Lack Lackkarten angefertigt und diese koloristisch vermessen (Phyma, Geometrien 45°/0° Schwarzkarte, 22,5°/22,5° Schwarzkarte, 45°/0° Weißkarte).
Die erfindungsgemäße Pigmentmischung zeigt eine farbneutrale silberne Interferenz (Farbstärke C = 3.20) mit sehr hohem Glanz (L-Wert: 84.15).

Vergleichsbeispiel 1: Herstellung einer farbneutralen Pigmentmischung mit einem farbstarken roten Interferenzpiament

Die Pigmentpulver aus a) und c) werden im Verhältnis 2:1 (grünlich-silber:rot) gemischt.
Von der Pigmentmischung werden nach Einarbeitung in Nitrocellulose-Lack Lackkarten angefertigt und diese koloristisch vermessen (Phyma, Geometrien 45°/0° Schwarzkarte, 22,5°/22,5° Schwarzkarte, 45°/0° Weißkarte).
Das Produkt zeigt eine farbneutrale silberne Interferenz (Farbstärke C = 3.09) mit einem geringeren Glanz als die erfindungsgemäße Mischung aus Beispiel 1 (L-Wert: 78.04).

Vergleichsbeispiel 2: Herstellung einer farbneutralen Pigmentmischung aus einem farbstarken roten und grünen Interferenzpigment

Die Pigmentpulver aus c) und d) werden im Verhältnis 1:1 gemischt.
Von der Pigmentmischung werden nach Einarbeitung in Nitrocellulose-Lack Lackkarten angefertigt und diese koloristisch vermessen (Phyma, Geometrien 45°/0° Schwarzkarte, 22,5°/22,5° Schwarzkarte, 45°/0° Weißkarte).
Das Produkt zeigt eine farbneutrale silberne Interferenz (Farbstärke C = 2.72) ohne Glanz (L-Wert: 59.17).

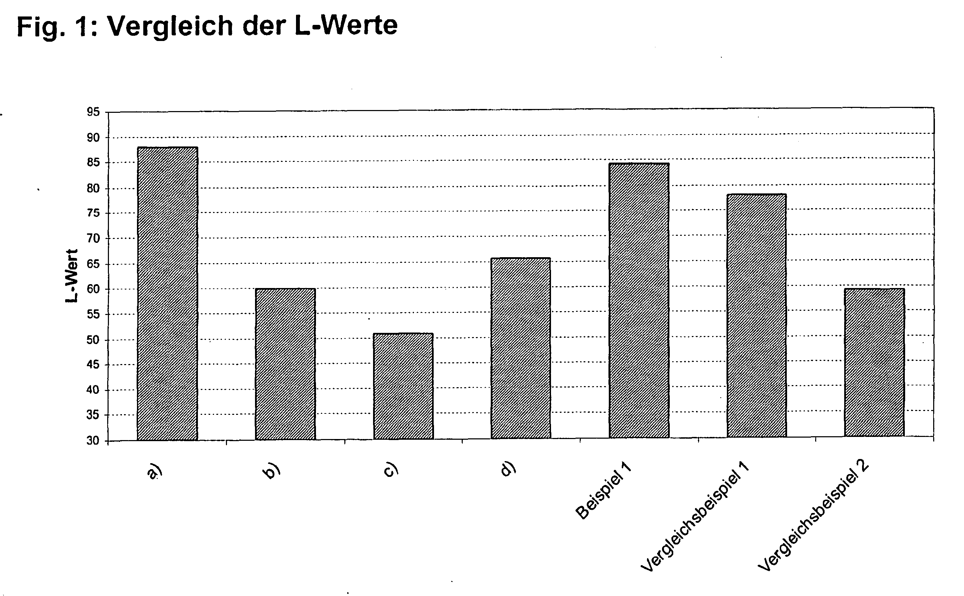

Figur 1 zeigt, dass erfindungsgemäße Pigmentmischungen einen höheren Glanz aufweisen als die gemäß den Vergleichsbeispielen erhaltenen Mischungen.

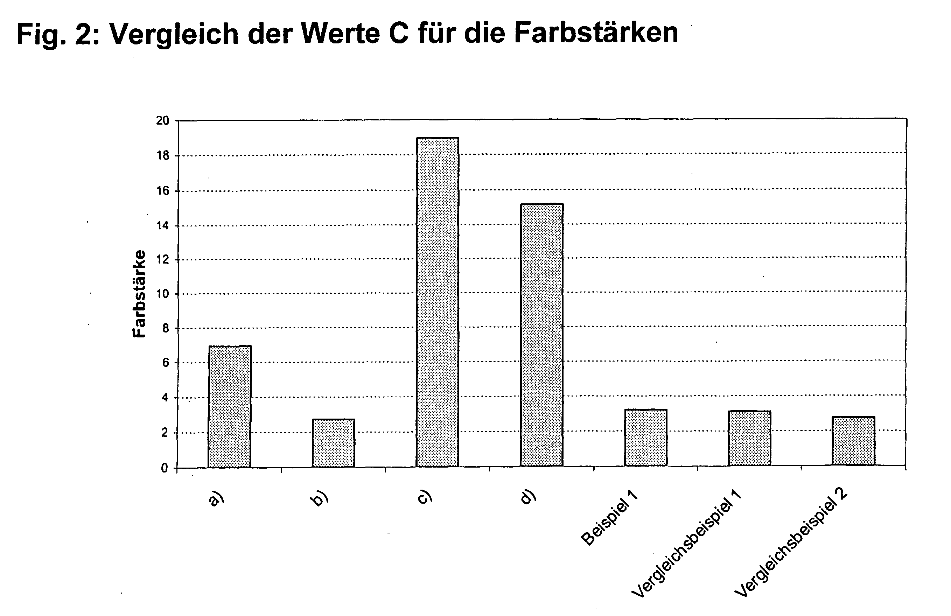

Figur 2 gibt einen vergleichenden Überblick über die Werte für die Farbstärke C der Pigmente a) - d) sowie der Mischung aus Beispiel 1 und den Vergleichsbeispielen. Durch erfindungsgemäße Kombination von farbschwachen silbernen Pigmenten mit farbschwachen komplementär-farbigen Pigmenten werden farbneutrale silberne Pigmentmischungen mit hohem Glanz erhalten.

**Patentansprüche**

1. Silberne und farbneutrale Pigmentmischung umfassend farbschwache silberne Interferenzpigmente A und farbschwache komplementär-farbige Interferenzpigmente B, wobei der Wert C für die Farbstärke der Pigmentmischung ≤ 4.0 ist und der Wert C für die Farbstärke der farbschwachen Interterenzpigmente A und B zwischen 4.0 und 10.0 liegt.

2. Pigmentmischung gemäß Anspruch 1, **dadurch gekennzeichnet, dass** die Interferenzpigmente A und B auf Trägermaterialien basieren, die mit einer oder mehreren Schichten enthaltend Metalloxide, Metalloxidhydrate, Metallsuboxide, Metalle, Metallfluoride, Metallnitride, Metalloxynitride und/oder Mischungen hieraus beschichtet sind.

3. Pigmentmischung gemäß Anspruch 2, **dadurch gekennzeichnet, dass** das Trägermaterial Titanoxide, synthetischen oder natürlichen Glimmer, Schichtsilikate, Glas, Siliziumdioxid, Eisenoxid und/oder Aluminiumoxid umfasst.

4. Pigmentmischung gemäß Anspruch 2 oder 3, **dadurch gekennzeichnet, dass** die Trägermaterialien mit alternierenden Schichten von Materialien mit hoher und niedriger Brechzahl beschichtet sind.

5. Pigmentmischung gemäß Anspruch 4, **dadurch gekennzeichnet, dass** die Materialien mit niedriger Brechzahl $SiO_2$, $SiO(OH)_2$, $Al_2O_3$, $AlO(OH)$, $B_2O_3$, $MgF_2$ und/oder Mischungen hieraus sind.

6. Pigmentmischung gemäß Anspruch 4, **dadurch gekennzeichnet, dass** die Materialien mit hoher Brechzahl $TiO_2$, $ZrO_2$, $ZnO$, $SnO_2$, $BiOCl$ und/oder Mischungen hieraus sind.

7. Pigmentmischung gemäß einem der Ansprüche 1 bis 6, **dadurch gekennzeichnet, dass** die Interferenzpigmente A und B weiterhin mit einer zusätzlichen organischen Beschichtung als äußere Schicht versehen sind.

8. Verwendung von Pigmentmischungen gemäß Anspruch 1 in Kosmetika, Lacken, Farben, Kunststoffen, Folien, im Sicherheitsdruck, in Sicherheitsmerkmalen in Dokumenten und Ausweisen, zur Lasermarkierung, zur Saatguteinfärbung, zur Lebensmitteleinfärbung oder in Arzneimittelüberzügen sowie zur Herstellung von Pigmentpräparationen und Trokkenpräparaten.

9. Kosmetika, Lacke, Farben, Kunststoffe, Folien, Dokumente und Ausweise, Saatgut, Lebensmittel oder Arzneimittelüberzüge Pigmentpräparationen und Trockenpräparate enthaltend Pigmentmischungen gemäß Anspruch 1.

**Claims**

1. Silvery and neutral-coloured pigment mixture comprising weakly coloured silvery interference pigments A and weakly coloured interference pigments B of a complementary colour, where the value C for the chroma of the pigment mixture is $\leq 4.0$ and the value C for the chroma of the weakly coloured interference pigments A and B is between 4.0 and 10.0.

2. Pigment mixture according to Claim 1, **characterised in that** the interference pigments A and B are based on support materials which are coated with one or more layers comprising metal oxides, metal oxide hydrates, metal suboxides, metals, metal fluorides, metal nitrides, metal oxynitrides and/or mixtures thereof.

3. Pigment mixture according to Claim 2, **characterised in that** the support material comprises titanium oxides, synthetic or natural mica, phyllosilicates, glass, silicon dioxide, iron oxide and/or aluminium oxide.

4. Pigment mixture according to Claim 2 or 3, **characterised in that** the support materials are coated with alternating layers of materials of high and low refractive index.

5. Pigment mixture according to Claim 4, **characterised in that** the materials of low refractive index are $SiO_2$, $SiO(OH)_2$, $Al_2O_3$, $AlO(OH)$, $B_2O_3$, $MgF_2$ and/or mixtures thereof.

6. Pigment mixture according to Claim 4, **characterised in that** the materials of high refractive index are $TiO_2$, $ZrO_2$, $ZnO$, $SnO_2$, $BiOCl$ and/or mixtures thereof.

7. Pigment mixture according to one of Claims 1 to 6, **characterised in that** the interference pigments A and B are furthermore provided with an additional organic coating as outer layer.

8. Use of pigment mixtures according to Claim 1 in cosmetics, paints, coatings, plastics, films, in security printing, in security features in documents and identity cards, for laser marking, for colouring seed, for colouring foods or in medicament coatings and for the preparation of pigment compositions and dry preparations.

9. Cosmetics, paints, coatings, plastics, films, documents and identity cards, seed, foods or medicament coatings, pigment compositions and dry preparations comprising pigment mixtures according to Claim 1.

**Revendications**

1. Mélange de pigments de couleur neutre et argentés comprenant des pigments d'interférence A argentés faiblement colorés et des pigments d'interférence B faiblement colorés de couleur complémentaire, où la valeur C pour le chroma du mélange de pigments est $\leq 4,0$ et la valeur C pour le chroma des pigments d'interférence A et B faiblement colorés est comprise entre 4,0 et 10,0.

2. Mélange de pigments selon la revendication 1, **caractérisé en ce que** les pigments d'interférence A et B sont basés sur des matériaux supports qui sont revêtus d'une ou plusieurs couches comprenant des oxydes métalliques, des oxydes métalliques hydratés, des sous-oxydes métalliques, des métaux, des fluorures métalliques, des nitrures métalliques, des oxynitrures métalliques et/ou des mélanges de ceux-ci.

3. Mélange de pigments selon la revendication 2, **caractérisé en ce que** le matériau support comprend des oxydes de titane, du mica synthétique ou naturel, des phyllosilicates, du verre, du dioxyde de silicium, de l'oxyde de fer et/ou de l'oxyde d'aluminium.

**4.** Mélange de pigments selon les revendications 2 ou 3, **caractérisé en ce que** les matériaux supports sont revêtus de couches alternées de matériaux d'indice de réfraction élevé et faible.

**5.** Mélange de pigments selon la revendication 4, **caractérisé en ce que** les matériaux d'indice de réfraction faible sont $SiO_2$, $SiO(OH)_2$, $Al_2O_3$, $AlO(OH)$, $B_2O_3$, $MgF_2$ et/ou des mélanges de ceux-ci.

**6.** Mélange de pigments selon la revendication 4, **caractérisé en ce que** les matériaux d'indice de réfraction élevé sont $TiO_2$, $ZrO_2$, $ZnO$, $SnO_2$, $BIOCl$ et/ou des mélanges de ceux-ci.

**7.** Mélange de pigments selon l'une des revendications 1 à 6, **caractérisé en ce que** les pigments d'interférence A et B sont pourvus en outre d'un revêtement organique supplémentaire comme couche externe.

**8.** Utilisation de mélange de pigments selon la revendication 1, dans des produits cosmétiques, des peintures, des revêtements, des matières plastiques, des films, dans l'impression de sécurité, dans des dispositifs de sécurité de documents et de papiers d'identité, pour le marquage au laser, pour la coloration de semences, pour la coloration d'aliments ou dans des enrobages de médicaments et pour la préparation de compositions pigmentaires et de préparations sèches.

**9.** Produits cosmétiques, peintures, revêtements, matières plastiques, films, documents et papiers d'identité, semences, aliments ou enrobages de médicaments, compositions pigmentaires et préparations sèches comprenant des mélanges de pigments selon la revendication 1.

Fig. 1: Vergleich der L-Werte

# Fig. 2: Vergleich der Werte C für die Farbstärken

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1213330 A
- DE 10331903 A
- EP 1422208 A
- EP 0632109 A
- US 5759255 A
- DE 4317019
- DE 3929423
- DE 3235017
- EP 0492223 A
- EP 0342533 A
- EP 0268918 A
- EP 0141174 A
- EP 0764191 A
- WO 9813426 A
- EP 0465805 A

**In der Beschreibung aufgeführte Nicht-Patentliteratur**

- Colorimetric evaluation of pearlescent pigments, Congress. **F. Hofmeister.** Mondial Couleur 85. 1985
- **R. Glausch ; M. Kieser ; R. Maisch ; G. Pfaff ; J. Weitzel.** Perlglanzpigmente. Curt R. Vincentz Verlag, 1996, 83